# EUROPÄISCHE PATENTSCHRIFT

(11) **EP 2 046 414 B1**
(45) Veröffentlichungstag und Bekanntmachung des Hinweises auf die Patenterteilung: **04.01.2017**
(21) Anmeldenummer: 07786114.4
(22) Anmeldetag: 17.07.2007
(51) Int. Cl.: A61M 1/10, A61M 1/12

(54) **IMPLANTIERBARES ZWEIKAMMERSYSTEM ZUM UNTERSTÜTZEN DES LINKEN HERZVENTRIKELS**
IMPLANTABLE TWO-CHAMBER SYSTEM FOR SUPPORTING THE LEFT VENTRICLE OF THE HEART
SYSTÈME À DEUX CHAMBRES IMPLANTABLE DESTINÉ À SUPPORTER LE VENTRICULE GAUCHE DU COEUR

(30) Priorität: 28.07.2006 DE 102006035798
(43) Veröffentlichungstag der Anmeldung: 15.04.2009
(73) Patentinhaber: Deutsches Zentrum für Luft- und Raumfahrt e.V., 51147 Köln (DE)
(72) Erfinder: VODERMAYER, Bernhard, 82205 Gilching (DE); SCHMID, Thomas, 82347 Bernried (DE)
(74) Vertreter: dompatent von Kreisler Selting Werner - Partnerschaft von Patent- und Rechtsanwälten mbB
(86) Internationale Anmeldenummer: PCT/EP2007/006314
(87) Internationale Veröffentlichungsnummer: WO 2008/012007

(56) Entgegenhaltungen:
- EP-A- 0 272 445
- WO-A-93/17730
- DE-A1- 10 217 635

## Beschreibung

### Technisches Gebiet

Die Erfindung betrifft ein implantierbares Zweikammersystem zum Unterstützen des linken Herzventrikels mit einer Pumpeinrichtung bestehend aus zwei Pumpkammern und einer zwischen diesen angeordneter Druckplatte, die mittels einer Antriebseinheit zwischen zwei Endlagen hin und her bewegbar ist, wodurch Fluid in einer Pumpkammer komprimiert wird, während die andere befüllt wird.

Ein derartiges Zweikammersystem ist beispielsweise in DE 102 17 635 A1 beschrieben.

Mechanische Kreislauf-Unterstützungssysteme (VAD: Ventricular Assist Device) werden seit etwa 10 Jahren klinisch eingesetzt und stellen bei manifestierter Herzinsuffizienz die letzte Option für einen Lebenserhalt dar. Herzunterstützungssysteme übernehmen einen Teil der Pumparbeit und stabilisieren dadurch den Kreislauf, bis ein Spenderorgan zur Verfügung steht. Neuere Untersuchungen zeigen, dass sich unter dieser Therapie die Herzfunktion soweit bessern kann, dass eine Explantation des Systems ohne anschließende Herztransplantation möglich ist.

Künstliche Herzpumpen können an verschiedene Anforderungen angepasst werden und stehen den Kliniken ohne Wartefrist zur Verfügung; allerdings bergen sie auch Nachteile und Risiken. Insbesondere hat die Therapie hinsichtlich der eingesetzten Technik sowie der Verträglichkeit Grenzen. Das Blut kann durch die Pumparbeit geschädigt werden. Die Versorgung der momentan ausschließlich elektrisch betriebenen Systeme, die mittels Kabel durch die Bauchdecke mit der erforderlichen Energie versorgt werden, birgt ein hohes Infektionsrisiko für den Patienten. Niedrige Wirkungsgrade bedingen einen hohen Energieverbrauch und erwärmen dadurch das umliegende Gewebe. Die assistierte Zirkulation muss oft über Monate und Jahre aufrechterhalten werden. Die Systeme sind starken mechanischen Belastungen unterworfen. Da ein rascher Wechsel der Blutpumpen nicht möglich ist, müssen diese hohe Standfestigkeit und Sicherheit aufweisen.

Da Unterstützungssysteme implantiert werden und zudem großen mechanischen Belastungen ausgesetzt sind, müssen sie zuverlässig und möglichst körperverträglich sein. Probleme treten vor allem bei Dauerbelastung und damit einhergehender Materialermüdung sowie auch mit Herzklappen auf. Das mechanische Versagen der Prothesen führt zu verminderter Pumpleistung. Eine ungünstige Position führt zu Thrombenbildung, bevorzugt am Rand der Klappen, und reduziert dadurch den effektiven Öffnungsdurchmesser der Prothese. Die Anzahl der bewegten Teile wie Lager, Wellen, Nocken etc., stellt ein inhärentes Risiko dar; je geringer dieses Risiko ist, desto geringer ist auch die Ausfallswahrscheinlichkeit des Gesamtsystems.

Herzunterstützungssysteme, die nach dem Verdrängungsprinzip arbeiten, komprimieren die Pumpkammer mechanisch oder pneumatisch, was einen Ausgleich des verdrängten Volumens erforderlich macht. Einkammersysteme müssen das komprimierte Pumpvolumen durch Kanülen an die Hautoberfläche oder implantierte Volumenausgleichsbehälter (sog. Vent) kompensieren, was ein zusätzliches Risiko darstellt. Auch Einkammersysteme können nur für die Unterstützung eines Ventrikels eingesetzt werden.

Die Thrombenbildung stellt nach wie vor das Hauptproblem beim Einsatz artifizieller Blutpumpen dar. Es wird von einem direkten Zusammenhang zwischen der Strömung in den Pumpkammern, den dadurch auftretenden Scherkräften und der Bildung von Blutgerinnseln (sog. Clots) ausgegangen. Ablagerungen treten bevorzugt in Gebieten von Rezirkulation und beim Wiederanlegen abgelöster Strömung auf. Die Zerstörung von roten Blutkörperchen (Hämolyse) konnte dagegen insbesondere in Gebieten mit hohen Scherkräften nachgewiesen werden.

Bisher eingesetzte, bogenförmig ausgeführte Pumpkammern ermöglichen ein kompaktes Design, führen jedoch zu einer Rezirkulation und Stagnation der Strömung, wodurch die Bildung von Thromben begünstigt wird. Ausgeprägte Wirbelgebiete, wie sie auch in den meist runden Kammern bekannter Systeme beobachtet werden, mindern zudem den Wirkungsgrad der Pumpen. Die Kompression mit einer rigiden Druckmembran behindert die Strömung in den Pumpkammern nachhaltig und fördert die Bildung von Rezirkulationsgebieten und Ablagerungen. Zudem besteht die Gefahr, dass an ungünstig angeströmten Stellen hohe Scherkräfte entstehen, durch die das Blut geschädigt werden kann.

### Darstellung der Erfindung

Aufgabe der Erfindung ist es daher, die Pumpkammern eines implantierbaren Zweikammersystems so zu bemessen und auszulegen, dass die Fluidströmung in den Pumpkammern eine möglichst physiologische, dem menschlichen Herzen ähnliche Strömung ist. Gemäß der Erfindung ist diese Aufgabe bei einem implantierbaren Zweikammersystem gemäß dem Oberbegriff des Anspruchs 1 durch die Merkmale in dessen kennzeichnenden Teil gelöst. Vorteilhafte Weiterbildungen sind Gegenstand von unmittelbar oder mittelbar auf den Anspruch 1 rückbezogenen Ansprüchen.

Gemäß der Erfindung weist jede der beiden Pumpkammern des Zweikammersystems die Form eines ventrikelförmigen Beutels mit einem an einem Ende unmittelbar neben dem Auslass unter spitzem Winkel zum Auslass ausgebildeten Einlass auf. Ferner ist die Druckplatte außen in zwei Armen drehbar gelagert, welche an der Abtriebswelle einer Antriebseinheit beweglich gehaltert sind, so dass der auf der Abtriebswelle liegende Drehpunkt der Arme auf der Adaptern abgewandten Seite der Pumpkammern und damit außerhalb der Pumpkammern liegt.

Vorteilhafterweise liegt der Winkel zwischen Einlass und Auslass jeder Pumpkammer zwischen 30° und 60°. Ferner beträgt die maximale Breite im Einlass- und Auslassbereich jeder der Pumpkammern etwa 65 bis 72% der jeweiligen Pumpkammerlänge. Der übrige Teil der Pumpkammern ist schmaler ausgelegt. Darüber hinaus weist gemäß einer vorteilhaften Weiterbildung der Erfindung jede der ventrikelförmigen Pumpkammern anschließend an den Einlass zur Verringerung des Strömungswiderstands eine Ausbeulung mit einer Länge in der Größenordnung von 10 bis 15% der Pumpkammerlänge auf. Ferner ist gemäß der Erfindung der Einlass jeder Pumpkammer um etwa 10° zum sogenannten Pumpkammer-Boden geneigt. Damit die Strömungsgeschwindigkeit sowohl im Bereich des Einlasses als auch im Bereich des Auslasses möglichst wenig beeinflusst wird, beträgt der Durchmesser von Einlass und Auslass in etwa ein Drittel der Pumpkammerlänge.

Gemäß der Erfindung sind die beiden Pumpkammern derart optimiert, dass einströmendes Fluid minimal behindert wird und so ein optimales Strömungsprofil in der Kammer entstehen kann. Gemäß der Erfindung ausgestaltete Pumpkammern können somit nicht nur in einem Zweikammer-Herzunterstüzungssystem, sondern auch in Einkammerpumpen verwendet werden.

Bei den gemäß der Erfindung ausgebildeten Pumpkammern ist der Einsatz einer festen als Druckmembran wirkenden Druckplatte vorgesehen, die profiliert und im Randbereich gerundet ausgeführt ist. Die Pumpkammern können auch in Verbindung mit hydraulisch oder pneumatisch betriebenen Pumpen eingesetzt werden. Bei Einsatz der erfindungsgemäß ausgeführten Pumpkammern kann der Energieverbrauch der eingesetzten Pumpe reduziert werden, was wiederum vor allem bei implantierten Zweikammersystemen von Vorteil und wünschenswert ist.

Gemäß einer Weiterbildung der Erfindung beträgt die größte Dicke der profilierten Druckplatte im mittleren Bereich in etwa 10% der Pumpkammerlänge. Die Länge der an den Rändern gerundeten Druckplatte liegt bei etwa 73%, während die größte Breite bei etwa 66% der Pumpkammerlänge liegt. Damit sich an den Rändern der Pumpkammern ein Wulst bilden kann, kann die Druckplatte insgesamt schmaler als die jeweilige Pumpkammer bei maximaler Kompression ausgebildet sein.

Durch Einsatz der, wie vorstehend ausgeführt, profilierten und an den Rädern gerundeten Druckplatte in Verbindung mit der bei den ventrikelförmigen Pumpkammern ausgebildeten Ausbeulung ist ein moderater und gleichförmiger Übergang dadurch erreicht, dass ein zunächst parallele Anströmung aus einem Adapter in einem entsprechenden Winkel nach unten geführt wird und sich nicht von der Wand ablöst. Auf diese Weise entstehen keine Verluste durch eine Rezirkulation der Strömung.

Ferner ist durch die einlassseitig vorgesehene Ausbeulung in Verbindung mit der profilierten Druckplatte die Querschnittsfläche zwischen der Druckplatte und der Pumpkammerwandung vergrößert und damit auch an dieser Stelle der Strömungswiderstand erheblich geringer. Diese Maßnahme trägt somit in vorteilhafter Weise zur Ausbildung des vorstehend beschriebenen günstigen Strömungsprofils bei.

### Beschreibung der Zeichnungen

Nachfolgend wird die Erfindung anhand von Zeichnungen erläutert. Es zeigen:
- Fig.1a bis 1c: in Draufsicht, Schnittansicht sowie Seitenansicht eine Pumpkammer in Form eines ventrikelförmigen Beutels;
- Fig.2a bis 2c: in schematischer Darstellung ein Durchströmen einer ventrikelförmig ausgeführten Pumpkammer in unterschiedlichen Phasen;
- Fig.3a: eine Draufsicht auf eine Druckplatte und
- Fig.3b: eine Seitenansicht der auf einer Pumpkammer aufliegenden Druckplatte;
- Fig.4: eine Schnittansicht von Pumpkammern in einem Gehäuse;
- Fig.5: eine weitere Schnittdarstellung eines Gehäuses mit zwei Pumpkammern, und
- Fig.6: eine schematische Darstellung zur Führung und Lagerung der Druckplatte bezüglich einer Pumpkammer.

### Beschreibung der Erfindung

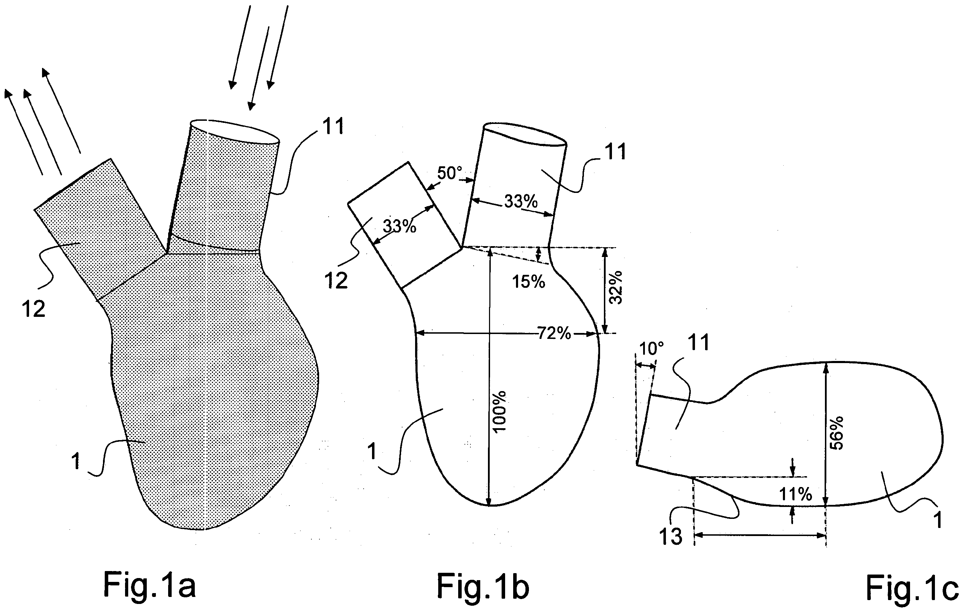

In Fig.1a und 1b ist in Draufsicht bzw. in einer Schnittansicht eine Pumpkammer 1 mit einem Einlass 11 und einem Auslass 12 wiedergegeben. Hierbei liegt der Öffnungswinkel zwischen dem unmittelbar bei dem Auslass 12 angeordneten Einlass 11 im Bereich von etwa 30 bis 60°. In Fig.1b ist ein Öffnungswinkel von 50° eingetragen.

Sowohl der Einlass 11 als auch der Auslass 12 haben verhältnismäßig große Querschnitte, deren Durchmesser bei etwa 33% der in Fig.1b mit 100% gekennzeichneten Länge der Pumpkammer 1 liegt. Die maximale Breite in Fig.1b etwa nach dem oberen Drittel (32% der Gesamtkammerlänge) liegt bei etwa 72%. Der anschließende Bereich von etwa zwei Drittel der gesamten Länge der Pumpkammer ist schmaler ausgelegt.

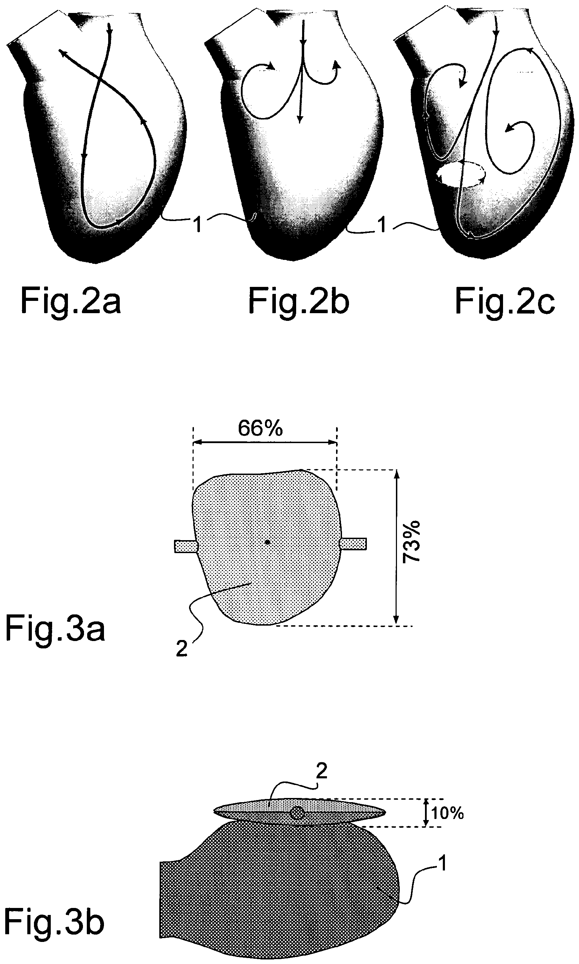

Wie der Schnittansicht der Fig.1c zu entnehmen ist, ist der Einlass 11 in der sogenannten Lateralebene um etwa 10° geneigt. Wie Fig.1c weiter zu entnehmen ist, weist die Pumpkammer 1 am "Boden" eine Ausbeulung 13 mit einer Länge in der Größenordnung von 11% der Pumpkammerlänge auf. Diese, einen großzügigen Radius aufweisende Ausbeulung 13 ist vorgesehen, damit der Querschnitt größer wird und somit die Strömungsgeschwindigkeit und der Strömungswiderstand niedriger werden. Wie in Fig.2b angedeutet, wird aufgrund der vorstehend beschriebenen Ausbildung der Pumpkammer 1 die Strömung von einem ausgeprägten linksdrehenden Wirbel bestimmt, wie er sich aufgrund der vorgegebenen Geometrie ausbildet. Generell beschreibt die Strömung in der Pumpkammer, wie in Fig.2a angedeutet, eine Art "8", deren oberer Kreis offen ist. Hierbei kreuzt eingeströmtes Fluid in der Systole, d.h. der Komprimierphase die Bahn während der Befüllungsphase, bevor das Fluid ausgeworfen wird. Hierbei wird das einströmende Fluid durch die zu Beginn einer Befüllungsphase noch vorhandene Deformation der Pumpkammer 1 an die außerseitige Kammerwand gelenkt, wie links in Fig.2c zu erkennen ist. Der rechte, entgegen dem Uhrzeigersinn drehende Wirbel in Fig.2b dominiert jedoch die Kammerströmung, so dass, wie in Fig.2c verdeutlicht ist, alle Bereiche der Pumpkammer 1 optimal durchspült werden. Durch eine strichpunktiert wiedergegebene, mit Pfeilen versehene Ellipse im unteren Bereich der Pumpkammer 1 ist der vorstehend bereits erwähnte gegen den Uhrzeigersinn drehende Wirbel nochmals hervorgehoben.

Wie bereits erwähnt, ist zur Optimierung der Strömung die Druckplatte 2 profiliert ausgeführt und an den Rädern abgerundet. Bei einer größten Dicke in der Mitte von etwa 10% bis 15% der Pumpkammerlänge beträgt, wie der Ansicht in Fig.3a zu entnehmen ist, die Länge der Druckplatte 2 etwa 73% der Länge der Pumpkammer; die größte Breite der Druckplatte liegt bezogen auf die Länge der Pumpkammer bei etwa 66%.

Die Druckplatte 2 kann schmaler ausgebildet sein als die Pumpkammer bei maximaler Kompression. Auf diese Weise kann sich, wie ebenfalls bereits ausgeführt, an den Rändern ein Wulst bilden, und das flexible Wandmaterial der Pumpkammer 1 kann ausweichen.

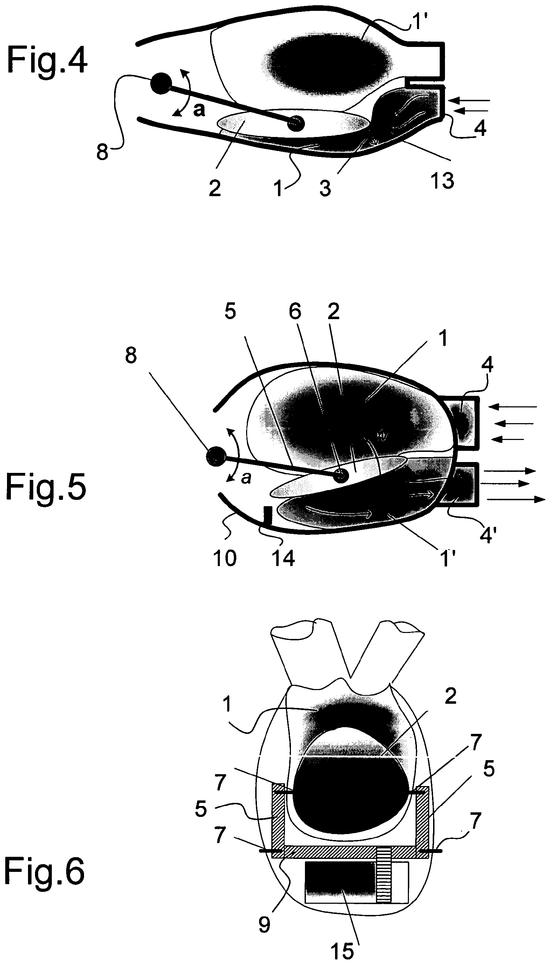

Aufgrund der profilierten Druckplatte 2 und der vorstehend beschriebenen Ausbeulung 13 ist in Fig.4 ein ausgesprochen moderater und gleichförmiger Übergang geschaffen. Die durch zwei waagrechte Pfeile angedeutete, zunächst parallele Strömung aus einem schematisch angedeuteten Zuflussadapter 4 wird unter einem durch gewellte Linien mit Pfeilspitzen angedeuteten Winkel in Fig.4 nach unten so geleitet, dass sich das Fluid nicht von der Wand ablöst. Damit entstehen auch keine Verluste durch eine Rezirkulation der Strömung. Wie bereits ausgeführt, wird durch die Ausbeulung 13 sowie durch die profilierte Druckplatte 2 eine mit 3 bezeichnete Querschnittsfläche vergrößert und somit der Strömungswiderstand an dieser Stelle erheblich verringert, so dass insgesamt ein günstiges Strömungsprofil erhalten wird.

In Fig.4 ist in etwa der Zeitpunkt dargestellt, zu welchem die Kompression des Fluids durch die Druckplatte 2 beendet ist und die durch mit Pfeilspitzen versehene Linien angedeutete Füllphase der Pumpkammer 1 gerade begonnen hat.

Beispielsweise in der eingangs angeführten DE 102 17 635 A liegen Arme, über welche eine dort vorgesehene Druckplatte von einer Antriebseinheit angetrieben und bewegt wird, innerhalb der Druckplatte; dies ist jedoch nachteilig, da die Arme immer wieder mit den Pumpkammerwänden in Berührung kommen und diese gegebenenfalls beschädigen.

Um dies zu vermeiden, sind gemäß der Erfindung, wie der schematischen Darstellung der Fig.6 zu entnehmen ist, Arme 5 außerhalb der Druckplatte 2 angeordnet und mit dieser durch Lager 6 (Fig.5) oder Zapfen 7 (Fig.6) schwenkbar verbunden. Ein auf der Abtriebswelle 9 einer nur schematisch angedeuteten Antriebseinheit 15 liegender Drehpunkt 8 der Arme 5 befindet sich auf der den Adaptern 4 und 4' abgewandten Seite der Pumpkammern 1 und 1' und damit außerhalb dieser Pumpkammern.

Durch eine Schwenkbewegung der Arme 5 um den Drehpunkt 8 (entsprechend einem mit Pfeilspitzen versehenen Bogen a, Fig.5) wird durch die Druckplatte 2 auf die Wände der Pumpkammern 1 und 1' und damit auf das in diesen Kammern vorhandene Fluid entsprechender Druck ausgeübt. Dabei führt die schwenkbar gelagerte Druckplatte 2 eine walkende Bewegung aus, wie durch einen mit Pfeilspitzen versehenen Bogen w in Fig.5 angedeutet ist. Durch diese walkende Bewegung wird das Fluid während der Komprimierphase zusätzlich in Richtung des Adapters 4' (Auslass) gedrückt, wie durch mit Pfeilen versehene gewellte Linien in Fig.5 angedeutet ist.

Ein einseitiges Verkippen der Druckplatte 2, das beim seitlichen Eingriff und ungünstigen Druckverhältnissen beobachtet wurde, wird dadurch nicht nur vermieden, sondern es ist für einen erheblich verbesserten Auswurf des Fluids gesorgt.

Um die walkende Bewegung gegen Ende einer Komprimierphase noch zu verstärken, kann in einem angedeuteten Gehäuse 10 jeweils ein im äußeren Randbereich der schwenkbar gelagerten Druckplatte 2 angeordneter Endanschlag 14 vorgesehen sein. Gleichzeitig wird durch den Endanschlag 14 verhindert, dass bei dem Auslenken der Arme 5 die Wände der Pumpkammern 1, 1' miteinander in Berührung kommen. Die beiden Pumpkammern 1, 1' sind derart angeordnet, dass erst dann, wenn die eine Pumpkammer vollständig entlastet ist, in der anderen Pumpkammer das Fluid komprimiert wird.

### Bezugszeichenliste

- 1, 1': Pumpkammer
- 2: Druckplatte
- 3: Querschnittsfläche
- 4, 4': Adapter
- 5: Arme
- 6: Lager
- 7: Zapfen
- 8: Drehpunkt
- 9: Abtriebswelle
- 10: Gehäuse
- 11: Einlass
- 12: Auslass
- 13: Ausbeulung
- 14: Endanschlag
- 15: Antriebseinheit

## Patentansprüche

1. Implantierbares Zweikammersystem zum Unterstützen des linken Herzventrikels, mit einer Pumpeinrichtung bestehend aus zwei Pumpkammern und einer zwischen diesen angeordneten 5 Druckplatte, die mittels einer Antriebseinheit zwischen zwei Endlagen hin und her bewegbar ist, wodurch Fluid in der einer Pumpkammer komprimiert wird, während gleichzeitig die andere Pumpkammer befüllt wird, **dadurch gekennzeichnet, dass** jede der beiden Pumpkammern (1, 1') die Form eines ventrikelförmigen Beutels mit einem an einem Ende unmittelbar neben einem Auslass (12) unter spitzem Winkel (a) zum Auslass ausgebildeten Einlass (11) aufweist, und dass die Druckplatte (2) außen in zwei Armen (5) drehbar gelagert ist, welche an der Abtriebswelle (9) einer Antriebseinheit (15) beweglich gehaltert sind, so dass der auf der Abtriebswelle (9) liegende Drehpunkt (8) der Arme (5) auf der dem Einlass (11) und dem Auslass (12) abgewandten Seite der Pumpkammern (1, 1') und damit außerhalb dieser Pumpkammern (1, 1') liegt.

2. Zweikammersystem nach Anspruch 1, **dadurch gekennzeichnet, dass** der spitze Winkel zwischen Einlass (11) und Auslass (12) jeder der Pumpkammern (1, 1') zwischen 30° und 60° liegt.

3. Zweikammersystem nach Anspruch 1, **dadurch gekennzeichnet, dass** die maximale Breite im Einlass- und Auslass-Bereich jeder der Pumpkammern (1, 1') etwa 65 bis 72% der jeweiligen Pumpkammerlänge beträgt, und die Pumpkammern (1, 1') in dem daran anschließenden Bereich schmaler ausgelegt sind, wobei die Pumpkammerlänge vom Ende des Einlasses (11) bis zum gegenüberliegenden Ende der Pumpkammer (1, 1') gemessen wird.

4. Zweikammersystem nach Anspruch 1, **dadurch gekennzeichnet, dass** der Einlass jeder Pumpkammer um etwa 10° zum Pumpkammer-Boden geneigt ist.

5. Zweikammersystem nach Anspruch 1, **dadurch gekennzeichnet, dass** der Durchmesser von Einlass (11) und Auslass (12) in etwa ein Drittel der Pumpkammerlänge beträgt.

6. Zweikammersystem nach Anspruch 1, **dadurch gekennzeichnet, dass** die Druckplatte (2) etwas schmaler ist als eine Pumpkammer (1) in komprimiertem Zustand.

7. Zweikammersystem nach Anspruch 1, **dadurch gekennzeichnet, dass** die Druckplatte (2) profiliert und im Randbereich gerundet ausgeführt ist.

8. Zweikammersystem nach Anspruch 1, 6 oder 7, **dadurch gekennzeichnet, dass** die maximale Dicke der Druckplatte (2) im mittleren Bereich etwa 5 bis 15% der Pumpkammerlänge sowie Breite und Länge der Druckplatte (2) etwa zwei Drittel der Länge der Pumpkammer (1) betragen.

9. Zweikammersystem nach Anspruch 1, 6, 7 oder 8 **dadurch gekennzeichnet, dass** die Druckplatte (2) im äußeren Bereich weniger steif als im mittleren Bereich ausgelegt ist.

10. Zweikammersystem nach Anspruch 1, **dadurch gekennzeichnet, dass** innerhalb eines die Pumpkammern (1, 1') umgebenden Gehäuses (10) jeweils ein im äußeren Randbereich der schwenkbar gelagerten Druckplatte (2) angeordneter Endanschlag (14) vorgesehen ist.

## Claims

1. An implantable two-chamber system for supporting the left ventricle of the heart, comprising a pumping device consisting of two pumping chambers and a pressure plate arranged therebetween, which pressure plate is adapted to be reciprocated between two end positions by means of a drive unit, whereby fluid is compressed in the one pumping chamber, whereas the other pumping chamber is filled at the same time, **characterized in that** each of both pumping chambers (1, 1') has the shape of a ventricular bag with an inlet (11) formed at one end immediately beside an outlet (12) and under an acute angle (a) with the outlet, and
that the pressure plate (2) is rotatably supported externally in two arms (5) that are movably retained at the output shaft (9) of a drive unit (15) so that the centre of motion (8) of the arms (5), which lies on the output shaft (9), is located on the side of the pumping chambers (1, 1') averted from the inlet (11) and the outlet (12) and thus outside these pumping chambers (1, 1').

2. The two-chamber system of claim 1, **characterized in that** the acute angle between the inlet (11) and the outlet (12) of each of the pumping chambers (1, 1') is between 30° and 60°.

3. The two-chamber system of claim 1, **characterized in that** the maximum width in the inlet and outlet region of each of the pumping chambers (1, 1') is about 65 to 72% of the respective pumping chamber length, and that the pumping chamber (1, 1') is narrower in the region adjacent the same, wherein the pumping chamber length is measured from the end of the inlet (11) to the opposite end of the pumping chamber (1, 1').

4. The two-chamber system of claim 1, **characterized in that** the inlet of each pumping chamber is inclined by about 10° to the pumping chamber bottom.

5. The two-chamber system of claim 1, **characterized in that** the diameter of the inlet (11) and the outlet (12) is about one third of the pumping chamber length.

6. The two-chamber system of claim 1, **characterized in that** the pressure plate (2) is slightly narrower than a pumping chamber (1) in the compressed state.

7. The two-chamber system of claim 1, **characterized in that** the pressure plate (2) is profiled and rounded at the edge portion.

8. The two-chamber system of claim 1, 6 or 7, **characterized in that** the maximum thickness of the pressure plate (2) in the central portion is about 5 to 15% of the pumping chamber length and the width and length of the pressure plate (2) amount to about two thirds of the length of the pumping chamber (1).

9. The two-chamber system of one of claims 1, 6, 7 or 8, **characterized in that** the pressure plate (2) is less rigid in the outer portion than in the central portion.

10. The two-chamber system of claim 1, **characterized in that**, within a housing (10) surrounding the pumping chambers (1, 1') a respective end stop (14) is provided in the outer edge portion of the pivotably mounted pressure plate (2).

## Revendications

1. Système à deux chambres implantable destiné à supporter le ventricule gauche du coeur, avec un dispositif de pompage comprenant deux chambres de pompage et une plaque de pression disposée entre celles-ci qui est déplaçable entre deux positions finales à l'aide d'une unité d'entraînement, ce par quoi du fluide est comprimé dans une des chambres de pompage alors que, simultanément, l'autre chambre de pompage est remplie,
**caractérisé en ce que** chacune des chambres de pompage (1, 1') présente la forme d'un sac ventriculaire avec une entrée (11) formée à une extrémité immédiatement à côté d'une sortie (12) sous un angle aigu (a) par rapport à la sortie, et **en ce que** la plaque de pression (2) est montée pivotante extérieurement sur deux bras (5) qui sont supportés de manière mobile sur un arbre de sortie (9) d'une unité d'entraînement (15), si bien que le point pivot (8) des bras (5) situé sur l'arbre de sortie (9) soit situé sur le côté des chambres de pompage (1, 1') éloigné de l'entrée (11) et de la sortie (12).

2. Système à deux chambres selon la revendication 1, **caractérisé en ce que** l'angle aigu entre l'entrée (11) et la sortie (12) de chacune des chambres (1, 1') est compris entre 30° et 60°.

3. Système à deux chambres selon la revendication 1, **caractérisé en ce que** la largeur maximale dans la zone d'entrée et de sortie de chacune des chambres de pompage (1, 1') est d'environ 65 à 72 % de la longueur respective de chambre de pompage, et **en ce que** les chambres de pompage (1, 1') sont formées plus étroites dans la zone adjacente, la longueur de chambre de pompage étant mesurée de l'entrée (11) jusqu'à l'extrémité opposée de la chambre de pompage (1, 1').

4. Système à deux chambres selon la revendication 1, **caractérisé en ce que** l'entrée de chaque chambre de pompage est inclinée d'environ 10° par rapport au fond de la chambre de pompage.

5. Système à deux chambres selon la revendication 1, **caractérisé en ce que** le diamètre de l'entrée (11) et de la sortie (12) est d'environ un tiers de la longueur de chambre de pompage.

6. Système à deux chambres selon la revendication 1, **caractérisé en ce que** la plaque de pression (2) est un peu plus étroite qu'une chambre de pompage (1) en état comprimé.

7. Système à deux chambres selon la revendication 1, **caractérisé en ce que** la plaque de pression (2) est pourvue d'un profile et est arrondie dans une zone de bord.

8. Système à deux chambres selon la revendication 1, 6 ou 7, **caractérisé en ce que** l'épaisseur maximale de la plaque de pression (2) dans une zone centrale est d'environ 5 à 15 % de la longueur de chambre de pompage et que la largeur et la longueur de la plaque de pression (2) sont d'environ deux tiers de la longueur de la chambre de pompage (1).

9. Système à deux chambres selon la revendication 1, 6, 7 ou 8, **caractérisé en ce que** la plaque de pression (2) est moins rigide dans la zone extérieure que dans la zone centrale.

10. Système à deux chambres selon la revendication 1, **caractérisé en ce qu'**à l'intérieur d'un boîtier (10) entourant les chambres de pompage (1, 1'), une butée (14) est prévue respectivement qui est disposée dans la zone extérieure de bord de la plaque de pression (2) montée pivotante.
